# EUROPEAN PATENT APPLICATION

(11) **EP 3 901 266 A1**
(43) Date of publication of application: **27.10.2021**
(21) Application number: 20170926.8
(22) Date of filing: 22.04.2020
(51) Int. Cl.: C12N 15/67, C12P 21/00

(54) **SUPER-ENHANCERS FOR RECOMBINANT GENE EXPRESSION IN CHO CELLS**

(71) Applicant: LEK Pharmaceuticals d.d., 1526 Ljubljana (SI)
(72) Inventor: Vogelsang, Matja, 1526 LJUBLJANA (SI); Tuta, Nika, 1526 LJUBLJANA (SI); Gaser, Dominik, 1526 LJUBLJANA (SI)
(74) Representative: Ter Meer Steinmeister & Partner

(57) **Abstract**

The present invention belongs to the field of biotechnology, and specifically relates to recombinant gene expression.

The invention concerns a method of recombinant gene expression from a Chinese Hamster Ovary (CHO) cell, by using super-enhancer sequences for increased gene expression. Thus, the invention provides a method for producing an engineered CHO cell by introducing into the cell an exogenous nucleic acid molecule into or within 500 kb upstream or downstream of a super-enhancer as expression-enhancing sequence. The invention further provides an engineered CHO cell produced by the method. The invention further provides a method of producing a recombinant polypeptide. The invention is further directed to the use of a super-enhancer for transgene expression.

## Description

The present invention belongs to the field of biotechnology, and specifically relates to recombinant gene expression.

The invention concerns a method of recombinant gene expression from a Chinese Hamster Ovary (CHO) cell by using super-enhancer sequences for increased gene expression. Thus, the invention provides a method for producing an engineered CHO cell by introducing into the cell an exogenous nucleic acid molecule into or within 500 kb upstream or downstream of a super-enhancer as expression-enhancing sequence. The invention further provides an engineered CHO cell produced by the method. The invention further provides a method of producing a recombinant polypeptide. The invention is further directed to the use of a super-enhancer for transgene expression.

### Background of the Invention

Chinese Hamster Ovary (CHO) cells are currently the preferred mammalian cell line used for production of recombinant proteins, especially proteins used for therapeutic applications. The main reasons for the use of CHO cells for recombinant gene expression is that CHO cells are capable of adapting and growing in suspension culture, which is ideal for large scale production, and CHO cells can grow in serum-free culture in chemically defined media, which ensures reproducibility. In addition, CHO cells allow human-like post-translational modifications to recombinant proteins, such as glycosylation of glycoproteins, as well as correct three-dimensional folding of the recombinant proteins. Meanwhile, expression systems for recombinant protein production from CHO cells are well-established. However, despite the availability of various expression systems, the challenge of efficient gene transfer and stability of the integrated gene for high expression of a recombinant protein still exists.

Current development of production cell lines is most commonly based on random integration of the transgene into the genome, resulting in low-level recombinant protein production and tedious screening of hundreds of clones. The present invention focuses on two problems commonly encountered during recombinant protein production from CHO cells, low quantity of protein and genetic instability of CHO cell lines used for recombinant gene expression.

In order to obtain high quantities of recombinant protein it is commonly tried to introduce not only one copy of a so called expression cassette including an exogenous nucleotide for recombinant gene expression into the cell chosen for recombinant protein production, but to try to introduce several copies of the expression cassette into a cell and subsequently select those modified host cells which have the optimal high number of expression cassettes in order to express the maximal amount of the protein of interest (POI). This strategy has at least two drawbacks:
First, the more copies of the expression cassette are introduced into the cell the more likely it is that over time the sequences of these expression cassettes recombine with each other due to the similarity of their sequences, which promotes recombination. As a consequence, rearrangements of nucleotide sequences within the modified host cell result in an instable integrity and copy number of the transgene sequence in the modified host cell used for protein expression. This results in a lower recombinant protein expression of the modified cell over time. In the worst case these unwanted recombination processes result in altered sequences of the POI, thereby not only decreasing the recombinant protein expression rate, but also decreasing the quality, because the recombinant protein gets a mixture of different variants of the POI, for example truncated or mutated versions of the POI, or POI with duplicated domains and region, etc.

Second, it is commonly recognized that a high copy number of an expression cassette is no guarantee for a high expression rate of the POI. Likely, a too high number of the expression cassette results in some kind of overburden or overstrain of the molecular machinery needed for protein expression of the modified host cell and thereby the expression rate of the POI goes down once the copy number of the expression cassette within the modified host cell exceeds a certain threshold.

Different solutions to the above-described problems associated with available expression systems have been suggested in the art.

WO 2019/038338 A1 overcomes the problems of prior art expression systems by introducing several expression cassettes into a cell which expression cassettes all code for the same mature recombinant POI, but which expression cassettes have different nucleotide sequences. For example, the expression cassettes may have different promoters, different terminators, different signal sequences, etc. and the coding sequence of the POI may be the same in the expression cassettes, or may be different in the different expression cassettes, however the amino acid sequence of the POI is always the same.

WO2017/184183 A1 provides a cell that contains an exogenous nucleic acid sequence integrated at a specific site within an enhanced expression locus, wherein the exogenous nucleic acid sequence encodes a bispecific antigen-binding protein.

One of the most essential functions of the active genome is gene transcription. Regions of the DNA that are bound by proteins that control transcription are called regulatory regions, which include enhancers, activators, promoters and insulators. Gene expression is controlled by enhancers, which are cell-type specific and activate transcription from the core promoters of their target genes. A typical mammalian genome contains between 400,000 and 1.4 million putative enhancers and it is estimated that between 10,000 and 150,000 enhancers are typically active in any one cell type. Enhancers are generally a few hundred base pairs in length and have been identified by using a variety of high-throughput techniques, including DNase-seq, ATAC-seq (both detect nucleosome-free regions of the genome representing active regulatory regions) and ChIP-seq (detect specific histone modification features representing enhancers).

Recently, WA Whyte et al. (Cell 153, April 11, 2013, 307-319) reported the discovery of a new class of regulatory elements called "super-enhancers", which have been defined to describe domains consisting of clusters of transcriptional enhancers that are densely occupied by master regulator transcription factors and are responsible to drive high-level expression of key genes that define cell identity and control cell state. Super-enhancers span large regions of chromatin with unusually high levels of master transcription factors domains, significant amounts of H3K4me1 and H3K27Ac histone modification and significant amounts of Mediator (MED1) occupancy. These super-enhancers differ from typical enhancers in size, transcription factor density and content, ability to activate transcription and sensitivity to perturbation. Super-enhancers are proposed to regulate nearby genes at the distances of up to 100kb and even up to 1-10Mb in some cases.

### Summary of the Invention

The present invention is based on the identification of super-enhancers throughout the genome of the CHO production cell line. These super-enhancers (also designated herein as "expression-enhancing sequence") are identified to drive high-level expression of nearby genes. It has been discovered in the present invention that these super-enhancers can beneficially be used for high-level expression of nearby-integrated transgenes.

Thus, the basic principle of the present invention is to introduce into a CHO cell an expression cassette including a transgene of interest within or in close proximity to a super-enhancer domain in order to achieve a stable and high-level expression of the transgene and to thereby produce the recombinant polypeptide or recombinant protein encoded by the transgene in high amounts.

In particular, the inventors have identified super-enhancer sequences in the CHO genome, which represent hot-spots for rapid development of production cell line, capable of producing high amounts of recombinant protein. Using targeted integration, a transgene of interest (TOI) can be integrated within or in proximity to the identified regions in order to achieve high-level transgene expression, resulting in high-yield production of recombinant protein. Moreover, identified super-enhancer sequences can be used for the design of expression cassettes or expression vectors for stable and high-level expression of recombinant protein from CHO production cell lines or, in consistency with this concept, production cell lines of other rodent species.

Thus, in a first aspect the present invention provides a method of producing an engineered Chinese Hamster Ovary (CHO) cell, the method comprising: introducing into the CHO cell one or more of a construct for integration of an exogenous nucleic acid molecule into and/or within 500 kb upstream or downstream of an expression-enhancing sequence in the genome of the cell, the expression-enhancing sequence being at least 90% identical to a sequence selected from any one of SEQ ID NOs: 1-47.

In a further aspect, the present invention provides an engineered cell produced by the method as described in the first aspect.

In another embodiment, the present invention provides a method of producing a recombinant polypeptide, the method comprising:
(i) introducing into a CHO cell one or more of a construct for integration of an exogenous nucleic acid molecule into and/or within 500 kb upstream or downstream of an expression-enhancing sequence in the genome of the cell, the expression-enhancing sequence being at least 90% identical to a sequence selected from any one of SEQ ID NOs: 1-47, to produce an engineered cell,
(ii) culturing the engineered cell to recombinantly express the exogenous nucleic acid to produce a recombinant polypeptide encoded by the exogenous nucleic acid, and
(iii) isolating the recombinant polypeptide.

In another embodiment, the present invention provides the use of a nucleic acid sequence being at least 90% identical to a sequence selected from any one of SEQ ID NOs: 1-47 for transgene expression.

### Description of Figures

**Figure 1****:** Comparison of the expression levels of three groups of genes: 1) 40 genes within 100kb proximity to several identified super-enhancers; 2) 37 genes within 100 kb proximity to identified typical regulatory regions (e.g. enhancers or promoters); and 3) 40 randomly selected genes.
**Figures 2-11** show the results of a comparison experiment of recombinant expression levels in CHO cells after targeted transgene integration into super enhancer regions or within proximity (50-100 kb) to super enhancer regions versus random integration.

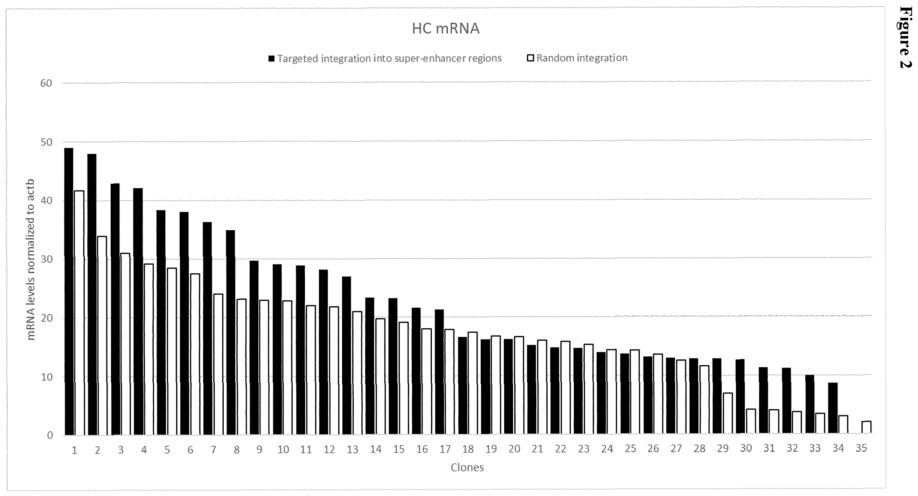

**Figure 2****:** Results of mRNA levels for heavy chain gene of IgG1.

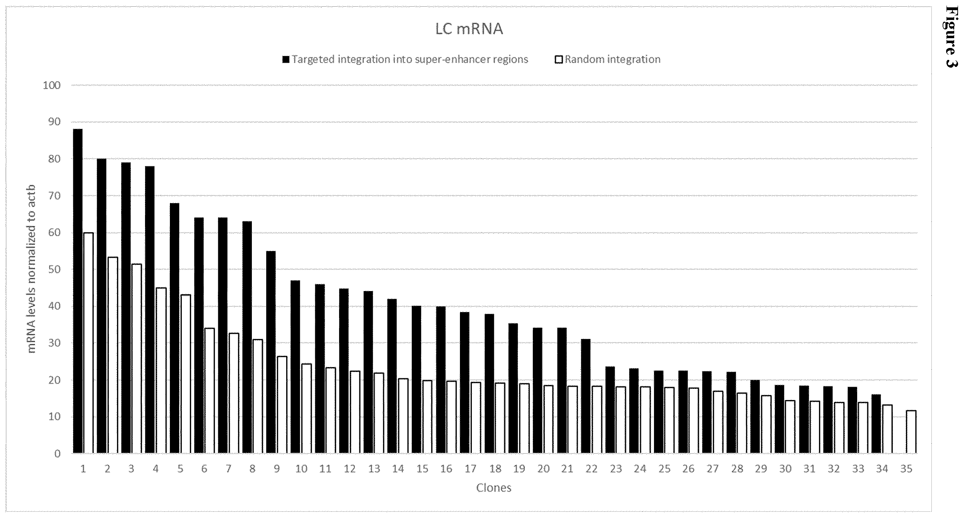

**Figure 3****:** Results of mRNA levels for light chain gene of IgG1.

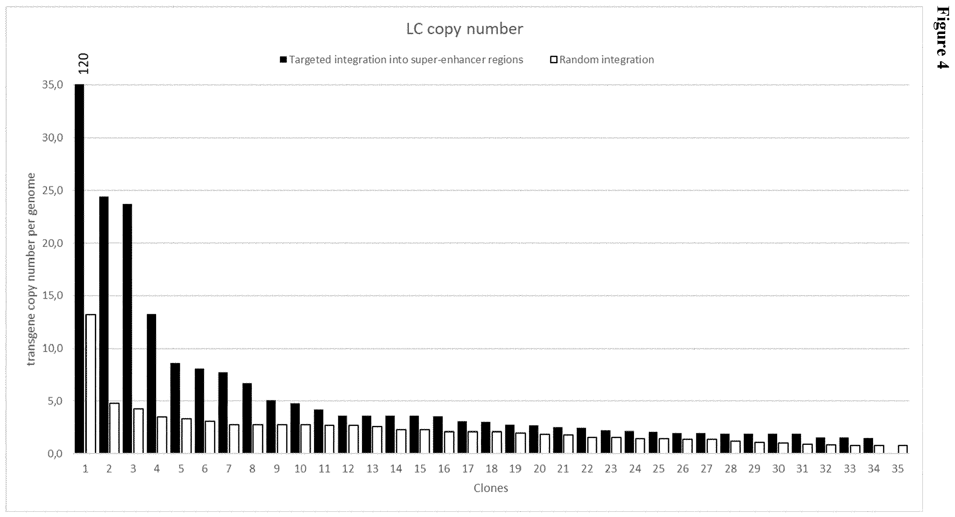

**Figure 4****:** Results of light chain gene copy number of IgG1.

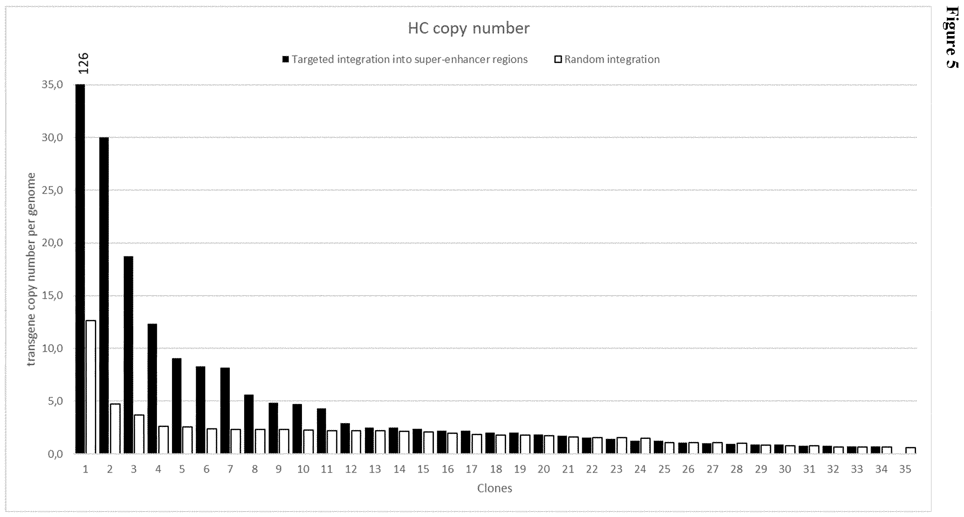

**Figure 5****:** Results of heavy chain gene copy number of IgG1.

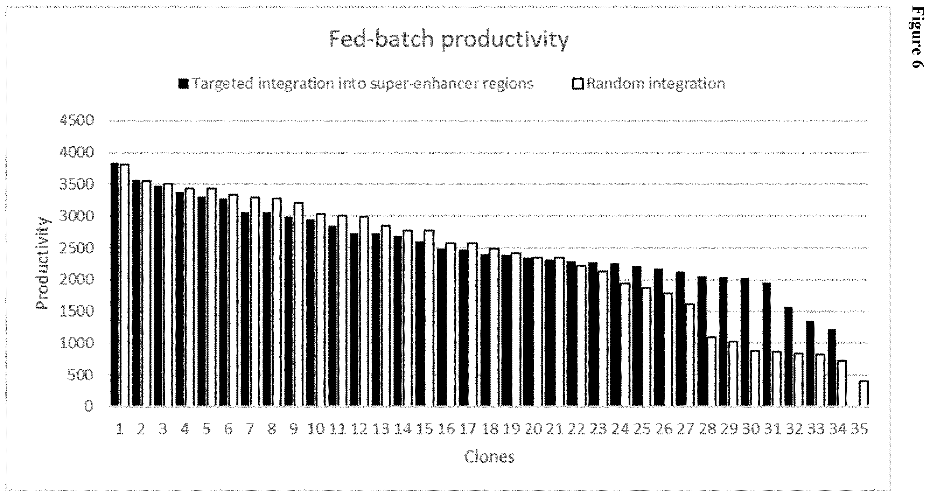

**Figure 6****:** Results of fed batch productivity of IgG1.

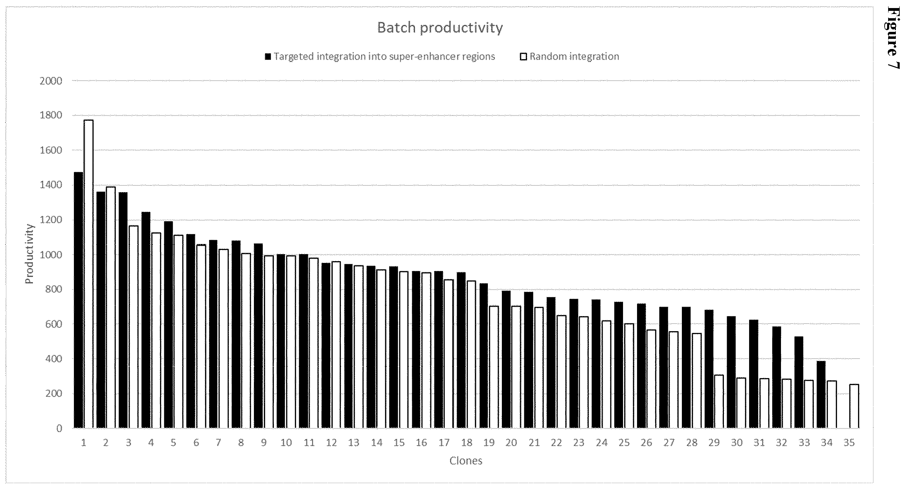

**Figure 7****:** Results of simple batch productivity of IgG1.

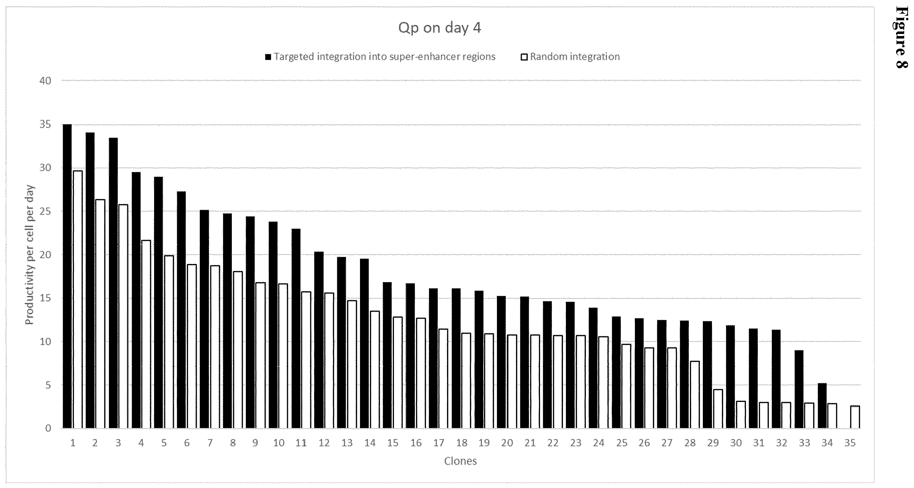

**Figure 8****:** Results of Cell-specific productivity from day 0 to day 4 of the simple batch proces s for IgG1 project.

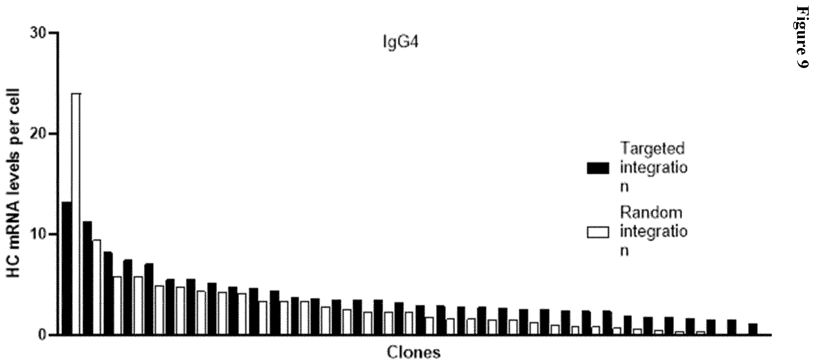

**Figure 9****:** Results of mRNA levels for heavy chain gene of IgG4.

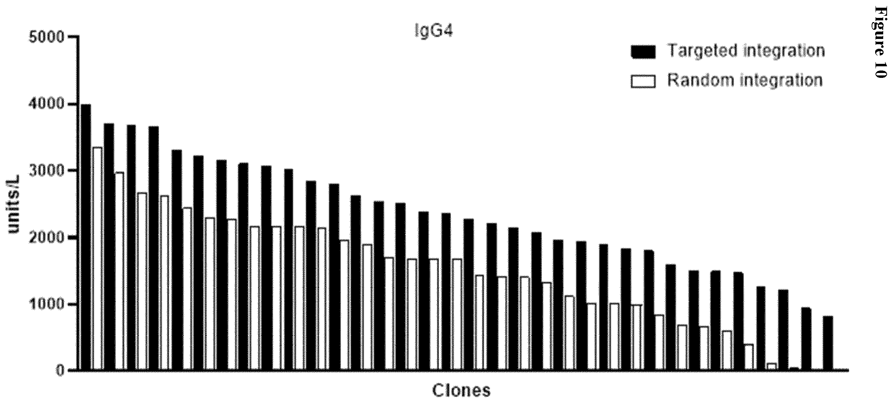

**Figure 10****:** Results of fed batch productivity of IgG4.

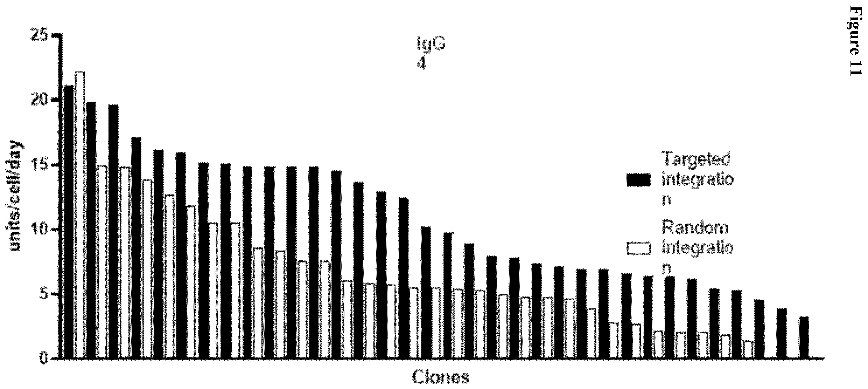

**Figure 11****:** Results of Cell-specific productivity from day 0 to day 4 of the simple batch process for IgG4 project

### Detailed Description of the Invention

In the first aspect, there is provided a method of producing an engineered rodent cell. The cell is preferably a hamster cell, most preferably a Chinese Hamster Ovary (CHO) cell. In other embodiments, the rodent includes the species mice, rats, squirrels, prairie dogs, chipmunks, chinchillas, porcupines, beavers, guinea pigs, gerbils and capybaras, preferably mice and rats. Hence, any reference to a CHO cell as provided herein is not intended to be limiting to the CHO cell, but instead the CHO cell can be any rodent cell as described above.

The Chinese Hamster Ovary (CHO) cell (also designated herein as "host cell") can be of any CHO cell line used in the art, such as CHO-K1, CHO-DXB11, CHO-S, CHO-DG44 and their derivatives. A commonly used cell line is DHFR⁻ CHO cell line which is auxotrophic for glycine, thymidine and hypoxanthine, and can be transformed to the DHFR⁺ phenotype using DHFR CDNA as an amplifiable dominant marker. One such known DHFR⁻ CHO cell line is DKXB11 (Urlaub et.al., (1980) Proc. Natl. Acad. Sci. USA 77:4216). Other cell lines developed for specific selection or amplification schemes will also be useful in the method of the present invention as described herein.

CHO cells and cell lines can be obtained from various sources such as the American Type Culture Collection (ATCC, Manassas, VA USA), the Deutsche Sammlung von Mikroorganismen und Zellkulturen GmbH (DSMZ; Braunschweig, Germany), or from commercial vendors such as Merck KGaA (Darmstadt, Germany), GE Healthcare (Buckinghamshire, Great Britain), or Thermo Fischer (Waltham, MA USA).

Unless otherwise stated, the CHO genome referenced herein refers to the Chinese hamster Assembly CHOK1S_HZDv1 from Eagle Genomics Ltd. dated 2017/06/30; GenBank assembly accession: GCA_900186095.1.

The method of the present invention, in particular, comprises the introduction of a construct for integration of an exogenous nucleic acid molecule into the CHO cell.

The term "exogenous nucleic acid molecule" refers to a nucleic acid molecule, typically a transgene, that is integrated at a site in a cell that is not the natural site for the nucleic acid molecule. For example, the nucleic acid molecule may naturally exist in the cell at a different site. Alternatively, the nucleic acid molecule may originate from a different cell. Typically, the exogenous nucleic acid encodes a polypeptide.

The construct, typically, is in the form of an expression cassette including a transgene of interest. "Expression cassette" means a polynucleotide sequence which typically comprises at least a promoter sequence, a start codon, a nucleic acid sequence, typically a transgene, coding for a protein of interest which is intended to be recombinantly expressed (POI), a stop codon and a terminator. The exogenous nucleic acid to be integrated into the genome of the CHO cell thus can include the transgene operably linked to a promoter that is functional in the engineered cell. The promoter sequence can be endogenous to the coding sequence. In some embodiments, the coding sequence is operably linked to a heterologous promoter sequence. Expression of the exogenous nucleic acid molecule can be further optimized using techniques known in the art. For example, the expression cassette may comprise additional regulatory and other sequences such as signal sequences, enhancers, introns, IRES- sequences, etc. The expression cassette might comprise additional parts, which parts are not directly needed for the expression of the POI, such as for example the origin of replication (ori), antibiotic resistance gene, or metabolic selection marker.

In the method of the present invention, the introduction of the construct into the CHO cell is typically achieved by transfection according to methods well-established in the art.

"Transfection" of a cell with a transgene of interest (typically meaning an expression cassette including the transgene) might result in transfected host cells (or transformed host cells, which is the same), wherein said host cells have integrated said transgene into their chromosomes. Said transgene might be integrated once or several times into said chromosome, preferably it is integrated several times into a chromosome, provided that integration is achieved at the integration site as defined herein.

Thus, in the method of the present invention, introducing the construct into the CHO cell leads to integration of the exogenous nucleotide sequence into the genome of the cell.

Typically, the integration of the exogenous nucleic acid molecule can be achieved by the CRISPR (Clustered Regulatory Interspaced Short Palindromic Repeats)/Cas9 method, TALEN (Transcription Activator-Like Effector Nuclease)-based method or ZFN (zinc-finger nuclease)-based method.

A homology recombination (HR) construct for insertion of an exogenous nucleic acid molecule at a target site within or near the super-enhancer described herein can be designed. The construct typically includes a first homology arm that is homologous to a sequence upstream of the target site and a second homology arm that is homologous to a sequence downstream of the target site. Each homology arm can include, for example, 200 to 1500 nucleotides (e.g., 200-250, 200-400, 250-500, 300-500, 400-600, 450-650, 500-800, 550-750, 650-900, 800-1000, 950-1200, or 1000-1500 nucleotides). The HR construct can further include multiple cloning sites between the two homology arms such that a gene to be inserted into the genome can be ligated into the construct. Alternatively, an HR construct containing the gene flanked by the two homologous sequences can be constructed using techniques known in the art, e.g., PCR. The HR construct can be used in a TALEN or CRISPR/Cas9 system to insert the nucleic acid molecule into the genome of the CHO cell.

TALEN and CRISPR/Cas9 methods both work by introducing a double-stranded DNA break in the genome at a target site. Based on the selected site, an HR construct harboring the nucleic acid molecule to be inserted at the target site can be designed and constructed.

CRISPR/Cas9 requires a gRNA specific to the targeted site and the endonuclease Cas9. The target site may be any sequence (about 20 nucleotides) that is unique compared to the rest of the genome and is immediately upstream of a Protospacer Adjacent Motif (PAM). Upon binding of the Cas9/gRNA complex to the target site, Cas9 cleaves the DNA. A skilled practitioner would be able to design a CRISPR/Cas9 construct directed at a target site.

TALEN utilizes a chimeric nuclease that contains an artificial DNA-binding domain of transcription activator-like effector (TALE) proteins and the catalytic domain of restriction endonuclease Fokl. As the code of DNA recognition by TALE proteins has been deciphered, an artificial DNA-binding domain for recognition of any DNA sequence can be designed. To minimize off-site effects, TALEN method can use a pair of chimeric nucleases that each recognizes a sequence on either side of the double-stranded DNA break site. A skilled practitioner would be able to design a TALEN construct directed at the selected site.

Zinc-finger nucleases (ZFNs) are artificial restriction enzymes generated by fusing a zinc finger DNA-binding domain to a DNA-cleavage domain. Zinc finger domains can be engineered to target specific desired DNA sequences, and this enables zinc-finger nucleases to target unique sequences within complex genomes. Each ZF set is linked to a cleavage domain, which must dimerize to cut DNA. Cleavage of the intended target gene can lead to disruption of its coding sequence by inaccurate repair through nonhomologous end joining. When a homologous donor DNA is introduced along with the ZFNs, it can be incorporated at the target by homologous recombination.

Preferably, integration of the exogenous nucleic acid molecule is achieved by the CRISPR /Cas9 method.

Integration of the exogenous nucleic acid molecule into the genome of a cell can be verified using methods known in the art. The engineered cells can be cultured under suitable conditions to express the nucleic acid molecule. Whether the engineered cell exhibits enhanced expression can also be determined using methods known in the art, e.g., ELISA, or RT-PCR.

The construct is integrated into the genome of the CHO cell at the target site (or "integration site", which is the same), which is located within the super-enhancer or within 500 kb upstream or downstream of the super-enhancer. The construct can be integrated into the genome of the cell at one target site as described herein or at more than one target site, such as two, three or multiple target sites. In this case, the construct is integrated into the genome of the CHO cell at the target sites, which are located within the super-enhancer and/or within 500 kb upstream or downstream of the super-enhancer(s).

The super-enhancer can be selected from a sequence that is at least 90%, preferably at least 91%, 92%, 93%, 94% further preferably at least 95%, 96%, 97% 98% or 99% identical to a sequence selected from any one of SEQ ID NOs: 1-47, or fragments thereof. In a particularly preferred embodiment, the super-enhancer is selected from any one of SEQ ID NOs: 1-47, or fragments thereof.
A suitable program for the determination of a level of identity is the nucleotide blast program (blastn) of NCBI's Basic Local Alignment Search Tool, using the "Align two or more sequences" option and standard settings
(http://blast.ncbi.nlm.nih.gov/Blast.cgi). As used herein, nucleotide sequence percent identity can be determined using a web based Clustal Omega, a multiple sequence alignment program with default parameters [Sievers and Higgins, Protein Sci. 2018 Jan; 27(1):135-1452018]. The percent identity value is a single numeric score determined for each pair of aligned sequences. It measures the number of identical residues ("matches") in relation to the length of the alignment.

It could be shown in the present invention that the super-enhancer sequences are highly conserved within rodent species, typically falling within 90% sequence identity, in particular within regulatory sequences, such as the one or more enhancer sequences clustered within a super-enhancer sequence. Therefore, even if not explicitly described in detail for any other rodent cell than a CHO cell, the invention as described herein is not restricted to a CHO cell but can be performed in any rodent cell due to high sequence conservation.

The group of sequences comprising the SEQ ID Nos: 1-47 define regions within the CHO genome, which are identified in the present invention as super-enhancers. The locations of these super-enhancer regions within the CHO genome are shown in Table 1.

**Table 1: List and location of CHO super-enhancer regions according to SEQ ID NO. 1-47 (Sequence locations referring to Chinese hamster Assembly CHOK1S_HZDv1 from Eagle Genomics Ltd. dated 2017/06/30; GenBank assembly accession: GCA_900186095.1.)**

| **SEQ ID NO.** | **Location (scaffold_ID:start-stop)** | **SEQ ID NO.** | **Location (scaffold_ID:start-stop)** |
|---|---|---|---|
| 1 | scaffold_0:127003236-127044012 | 25 | scaffold_16:33019037-33099603 |
| 2 | scaffold_0:127961999-127994763 | 26 | scaffold_16:34171408-34232652 |
| 3 | scaffold_1:5717943-5795504 | 27 | scaffold_19:38090598-38127487 |
| 4 | scaffold_1:96135496-96169600 | 28 | scaffold_202:25-78873 |
| 5 | scaffold_1:97016870-97051042 | 29 | scaffold_21:9452150-9482525 |
| 6 | scaffold_2:8990390-9071387 | 30 | scaffold_22:1971510-2008686 |
| 7 | scaffold_3:14201037-14232664 | 31 | scaffold_24:4856485-4948413 |
| 8 | scaffold_3:22455362-22480256 | 32 | scaffold_29:3784349-3827895 |
| 9 | scaffold_5:76073260-76113194 | 33 | scaffold_30:19104144-19104461 |
| 10 | scaffold_7:12057731-12105340 | 34 | scaffold_31:12785915-12827890 |
| 11 | scaffold_7:13607059-13619987 | 35 | scaffold_36:13274461-13303950 |
| 12 | scaffold_8:21329984-21392924 | 36 | scaffold_36:4881585-4935558 |
| 13 | scaffold_8:70124524-70197737 | 37 | scaffold_36:7709296-7803892 |
| 14 | scaffold_9:14918802-14981190 | 38 | scaffold_41:12017975-12063967 |
| 15 | scaffold_9:43934890-44003064 | 39 | scaffold_45:3901418-3950027 |
| 16 | scaffold_9:47068117-47112880 | 40 | scaffold_51:4864782-4934481 |
| 17 | scaffold_10:48106631-48149473 | 41 | scaffold_54:2219944-2303066 |
| 18 | scaffold_10:61021765-61110763 | 42 | scaffold_56:3494926-3554357 |
| 19 | scaffold_10:756101-843574 | 43 | scaffold_64:337435-383012 |
| 20 | scaffold_12:27781971-27828679 | 44 | scaffold_64:397437-420302 |
| 21 | scaffold_12:32514411-32671314 | 45 | scaffold_65:1291309-1351750 |
| 22 | scaffold_12:37165669-37207383 | 46 | scaffold_72:1526932-1571665 |
| 23 | scaffold_15:32528347-32556530 | 47 | scaffold_391:3899-38366 |
| 24 | scaffold_16:10909381-10994770 | | |

A target site for inserting an exogenous nucleic acid molecule can be located anywhere within or near (e.g., within 500 kb or within 100 kb upstream or downstream) the super-enhancer region. In some embodiments, the target site is located within 400 kb, 300 kb, 200 kb, 100 kb, 50 kb or 20 kb upstream or downstream of the super-enhancer. In a particularly preferred embodiment, the integration of the exogenous nucleic acid molecule is within 50 to 100 kb upstream or downstream of the super-enhancer. In a particularly preferred embodiment, the target site for inserting an exogenous nucleic acid molecule is located within the super-enhancer region.

In a further preferred embodiment, the exogenous nucleic acid molecule integrates at two or more integration sites in the genome of the cell, provided that at least one of the integration site is located anywhere within or near (e.g., within 500 kb upstream or downstream) the super-enhancer region as described-above.

Further, preferably, the exogenous nucleic acid molecule integrates at two, three or more integration sites located anywhere within or near (e.g., within 100 kb upstream or downstream) the super-enhancer region as described above.

More preferably, the exogenous nucleic acid molecule integrates at two, three or more integration sites located within the super-enhancer region as described above.

It has surprisingly been found in the present invention that these super-enhancer regions in the CHO genome can be used to produce an engineered CHO cell that highly expresses one or more exogenous nucleic acid molecules inserted within the genome of the engineered cell at the above-described target-sites within or near these super-enhancers.

Thus, in a further aspect, the present invention provides an engineered CHO cell produced by the method described above.

The engineered cell exhibits a higher (e.g., one or more folds) expression level of the exogenous nucleic acid molecule as compared to a control cell. A "control cell" can be a cell containing the same nucleic acid molecule inserted at a different site by random integration. For example, a control cell can be generated by randomly integrating the nucleic acid molecule into the genome of a CHO host cell. The CHO Consortium has also identified various potential genomic sites. A control cell can be produced by specifically inserting the nucleic acid molecule into one of these sites (Sofie A. O'Brien et al., Biotechnol. J. 2018, 13, 1800226, DOI: 10.1002/biot.201800226). The expression level can be measured at the mRNA level or protein level. As the engineered cell according to the invention or a control cell can contain more than one copy of the inserted nucleic acid molecule, the comparison can be normalized by determining the expression level per copy, such as comparing clones with single-copy integration.

Whether a target site within or near one of the super-enhancer enhances expression of the nucleic acid molecule can be determined by a skilled practitioner in the art. The precise location of the target site within or near the super-enhancer is not critical as long as the site can enhance expression and permit stable integration of a nucleic acid molecule. The site selection also depends on the genome editing technique used to insert the gene.

Hence, in a further aspect, the present invention is directed to the use of a super-enhancer as described herein and being at least 90% identical to a sequence selected from any one of SEQ ID NOs: 1-47 for transgene expression.

Further, as the super-enhancers can exert an expression-enhancing effect whether they are at their native genomic loci or at different loci, they can be included in expression vectors for transient expression of genes. For example, an expression vector can contain a gene and one or more of the super-enhancers as described herein. If more than one super-enhancer is included, they can be arranged in tandem with or without spacers between them. The vector can be introduced into a CHO cell to stably or transiently express the gene.

The engineered CHO cell described herein can be used in various commercial and experimental applications. In particular, the CHO cell can be employed for producing therapeutic polypeptides or proteins, such as antibodies, for example immunoglobulins or parts and fragments thereof.

Therefore, in another aspect the present invention provides a method of producing a recombinant polypeptide, the method comprising:
(i) introducing into a CHO cell a construct for integration of an exogenous nucleic acid molecule into or within 500 kb upstream or downstream of an expression-enhancing sequence in the genome of the cell, the expression-enhancing sequence being at least 90% identical to a sequence selected from any one of SEQ ID NOs: 1-47, to produce an engineered cell,
(ii) culturing the engineered cell to recombinantly express the exogenous nucleic acid to produce a recombinant polypeptide encoded by the exogenous nucleic acid, and
(iii) isolating the recombinant polypeptide.

Step (i) of the method is as described above with respect to the method of producing an engineered Chinese Hamster Ovary (CHO) cell according to the first aspect of the present invention.

Culturing the engineered cell to recombinantly express the exogenous nucleic acid to produce a recombinant polypeptide encoded by the exogenous nucleic acid in step (ii) and isolating the recombinant polypeptide in step (iii) can be performed by the skilled practitioner according to protocols well-established in the art.

### EXAMPLES

### Example 1: Identification of super-enhancers within the CHO genome.

Super-enhancers in the CHO genome were identified by applying ATAC-seq (Assay for Transposase Accessible Chromatin with high-throughput sequencing) approach on CHO cells by using a method as described in: JD Buenrostro et al., Nature Methods, Vol. 10(12), December 2013, 1213-1218. By this approach, open-chromatin regions throughout the CHO genome were identified, representing active regulatory regions. Regions were further analysed according to the protocol used for identification of super-enhancers from ChIP-seq data (JD Buenrostro et al. Curr Protoc Mol Biol., Vol. 109, 21.29.1-21.29.9.). A total of 47 active regulatory regions were identified genome-wide, representing putative super-enhancers (Table 1).

ATAC-seq was performed on three different parental CHO cell lines at day 3 and day 7 of the bioprocess, respectively, following the protocol described in Buenrostro et al. (2015) with minor adaptations as follows.

Cells were cultivated in shake flasks 250ml at 36.5°C, 10 % CO₂ with shaking at 150 rpm (R=25 mm). To prepare nuclei, 4e5 cells were spun at 500g for 5 min and washed with cold PBS. Cells were lysed using cold lysis buffer and immediately followed by transposition reaction as described in Buenrostro et al. protocol. The transposition reaction was carried out for 30 min at 37 °C and purified using a Qiagen MinElute Kit. Following purification, DNA was amplified using 1x NEBnext PCR master mix and 1,25µM of custom Nextera PCR primers Ad1_noMx and Ad1_2 (sequences are shown in Table 2), using the same PCR parameters as described in Buenrostro et al. protocol.

**Table 2: Primer sequences**

| | **Primer** | **Sequence** |
|---|---|---|
| **SEQ ID NO. 48** | Ad1_noMx | AATGATACGG CGACCACCGA GATCTACACT CGTCGGCAGC GTCAGATGTG |
| **SEQ ID NO. 49** | Ad1_2 | CAAGCAGAAG ACGGCATACG AGATTCGCCT TAGTCTCGTG GGCTCGGAGA TGT |

To reduce GC and size bias we monitored the PCR reaction using qPCR in order to stop amplification before saturation as advised from Buenrostro et al. Libraries were amplified for a total of 8-10 cycles.

In order to remove primer dimers and large > 1000 bp fragments, purification process was modified. Double-sided bead purification with AMPure XP magnetic beads was used instead of MinElute Kit. The quality of purified libraries was assessed with Bioanalyzer High Sensitivity DNA Analysis kit. Library quantitation was done with Qubit.

Paired-end sequencing was carried out on MiSeq (Illumina) with MiSeq reagent kit v2 (50 cycles) and data analysed using Array studio (Omicsoft) software. Reads were mapped to the reference genome CHOK1S_HZDv1 and peaks called using MACS algorithm with added criteria "Exclude multi-reads". The peaks were ranked based on peak score (Fold enrichment), clustered and intersected to identify super-enhancers. The ROSE (ranking of super-enhancer) algorithm is applied to successfully differentiate super-enhancers from typical enhancers and other regulatory regions.

In total, 47 super-enhancers were identified in majority of samples (i.e. 3 cell lines and 2 time-points of the bioprocess) and are listed in Table 1. Regions that are listed in Table 1 represent open and active regions in CHO genome that remained stably active during bioprocess and are common among parental CHO cell lines.

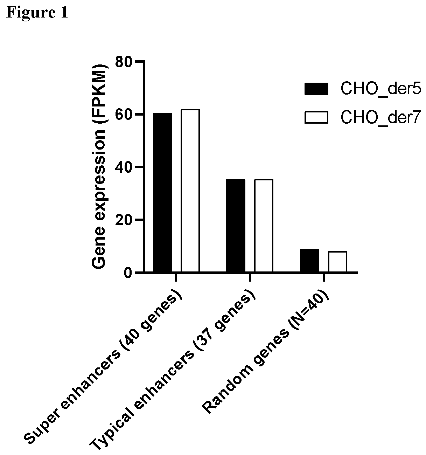

To confirm that the identified regions are super enhancers, massively parallel RNA-sequencing was carried out for two CHO derivative cell lines following standard RNA-seq procedures and expression levels for the following three groups of genes were extracted from available transcriptome data: 1) 40 genes within 100kb proximity to several identified super-enhancers; 2) 37 genes within 100kb proximity to identified typical regulatory regions (e.g. enhancers or promoters); and 3) 40 randomly selected genes. Average expression levels from the two in-house CHO cell lines were compared and results are presented in Figure 1.

Expression levels of genes found in 100kb proximity to super-enhancer regions were shown to be on average 7-fold higher, when compared to randomly selected genes.

In addition, super-enhancers are clusters of enhancers. To confirm this, the identified super enhancer regions were compared with publicly available data where Feichtinger et al. (Biotechnol Bioeng. 2016; 113(10); 2241-53) performed ChIP-seq experiment on CHO cells in order to define chromatin states, including promotors, enhancers, TSS, etc. Most of the regions identified overlapped with regions defined as enhancers by ChIP seq data, therefore further supporting the finding that these regions are super-enhancers.

### Example 2: Transgene expression under the control of super enhancers in the CHO cells

To further confirm the functionality of SE regions for increased expression of transgenes, the transgene sequences coding for human IgG4 and IgG1, were integrated into the target SE regions, and in the 50-100 kb proximity to the target SE regions, respectively. Three candidate SE regions were targeted including scaffold_12:37165669-37207383, scaffold_72:1526932-1571665 and scaffold_54:2219944-2303066. One region per clone was targeted. Productivity and mRNA levels were assessed and compared to stable cell lines generated by random integration of the same transgene sequence.

The position of the integration sites for IgG1 experiment are (near SE regions):
- scaffold_72:1135489-1135490
- scaffold_146:93341-93342 (159090 bp away from SE region scaffold_72:1526932-1571665)
- scaffold_12:3723885-373886

The position of the integration sites for IgG4 experiment are (within SE):
- scaffold_12:32605600-32605601 (referred to as 12-8 site)
- scaffold_12:32582184-32582185 (referred to as 12-10 site)
- scaffold_54:2259987-2259988 (referred to as 54-8 site)
- scaffold_72:1556270-1556271 (referred to as 72-5 site).

The procedure used to prepare stable cell lines is briefly described. First, cells were co-transfected with: 1) expression vector encoding for Cas9 nuclease and puromycin resistance gene (vector referred herein as Cas9 vector); 2) SwaI linearized expression vector encoding for heavy and light chain genes of an mAb molecule, as well as neomycin resistance gene (expression vector referred herein as mAb transgene); and 3) relevant PCR amplicon encoding for sgRNA (targeting a single genomic site for targeted integration) under U6 promoter, in molar ratios 1:1:6. Cell were incubated under standard conditions (36.5°C, 10 % CO2). Two days after transfections, cells were subjected to a three-step selection pressure consecutively using puromycin (5µg/mL), geneticin (0.8 mg/mL) and methotrexate (500 nM). Next, recovered stable cell pools were single-cell cloned using Cytena cell printer resulting in generation of stable monoclonal cell lines. The presence of targeted integration event was assessed in all generated stable cell lines using qPCR. Briefly, gDNA was extracted using KAPA Express Extract kit and four qPCR reactions per clone were performed targeting all four possible junction sites between CHO genome and integrated mAb transgene sequence (i.e. considering sense and anti-sense vector orientation and two junction sites per each vector orientation). PCR products exhibiting a single peak by the qPCR melting curve analysis suggested the presence of targeted integration event. Putative targeted integration events were further confirmed with amplicon-seq using illumina next generation sequencing platform (MiSeq).

Random integration clones (as a negative control) were generated by co-transfection with Cas9 vectors and SwaI linearized mAb transgene in molar ratio 1:1. The three step selection pressure and generation of monoclonal cell lines with random transgene integration were carried out following the same procedure as described above.

Volumetric and specific productivities of generated cell lines (targeted integration vs random integration) were analysed in batch and/or fed-batch processes. Titer determination was performed at days 4 and/or 14. Antibody titers in the cell culture supernatant were determined by Bio-Layer Interferometry technology using Octet system. mRNA levels of light and/or heavy chain genes were assessed at day 7 of the fed-batch process using droplet digital PCR (Bio-Rad).

Results are summarized in Tables 3 to 5 below and shown in Figures 2-11.

**Table 3: Targeted vs random integration summary (all relevant clones considered).**

| | **IgG1** | | | **IgG4** | | |
|---|---|---|---|---|---|---|
| | Targeted integration (average±std) | Random integration (average±std) | Fold difference (p-value) | Targeted integration (average±std) | Random integration (average±std) | Fold difference (p-value) |
| **N** | 34 | 35 | | 34 | 32 | |
| **Titer-simple batch** | 895±249 | 777±350 | 1.2 (0.1120) | NA | NA | |
| **Titer - fed-batch** | 2542±615 | 2320±983 | 1.1 (0.2635) | 2361±840 | 1586±825 | **1.5 (0.0004*)** |
| **LC mRNA levels** | 41.2±20.9 | 24.1±12.3 | **1.7 (0.0001*)** | NA | NA | |
| **HC mRNA levels** | 23.2±11.9 | 17.6±9.4 | **1.3 (0.0325)** | 3.7±2.2 | 2.6±2.1 | **1.4 (0.041)** |
| **Qp (day4) specific productivity** | 18.7±7.6 | 12.6±7.0 | **1.5 (0.0009*)** | 11.0±5.1 | 7.0±4.9 | **1.6 (0.0015*)** |

| | | | | | | |
|---|---|---|---|---|---|---|
| LC: low copy; HC: high copy; Qp, specific productivity - productivity per cell per day; bold, statistical significance *significant after multi-testing correction | | | | | | |

**Table 4: Targeted vs random integration summary (only clones with single-copy vector were considered**

| | **IgG1** | | | **IgG4** | | |
|---|---|---|---|---|---|---|
| | Targeted integration (average±std) | Random integration (average±std) | Fold difference (p-value) | Targeted integration (average±std) | Random integration (average±std) | Fold difference (p-value) |
| **N** | 12 | 11 | | 21 | 27 | |
| **Titer-simple batch** | 830±250 | 571±296 | **1.5 (0.0353)** | NA | NA | |
| **Titer - fed-batch** | 2292±413 | 1811±1010 | 1.3 (0.1651) | 1987±647 | 1569±825 | **1.3 (0.0627)** |
| **LC mRNA levels** | 34.5±20.3 | 16.9±2.6 | **2.0 (0.0093)** | NA | NA | |
| **HC mRNA levels** | 17.1±9.5 | 10.5±6.8 | **1.6 (0.0681)** | 3.8±2.9 | 2.5±2.1 | 1.5 (0.0874) |
| **Qp (day4) specific productivity** | 15.1±7.4 | 7.7±5.0 | **2.0 (0.0108)** | 8.8±4.4 | 6.2±4.1 | **1.4 (0.0388)** |

**Table 5: Comparison between separate targeted regions and random integration (only clones with single-copy vector were considered)**

| | **IgG4** | | | | |
|---|---|---|---|---|---|
| **Region*** | 12-10 (average±std) | 12-8 (average±std) | 54-8 (average±std) | 72-5 (average±std) | Random (average±std) |
| **N** | 6 | 2 | 6 | 7 | 27 |
| **Titer** - **fed-batch** | 2107±383 | 2864±336 | 2120±553 | 1520±676 | 1569±825 |
| **HC mRNA levels** | 3.2±2.6 | 6.3±1.1 | 2.9±0.7 | 4.3±4.2 | 2.5±2.1 |
| **Qp (day4) specific productivity** | 8.0±3.7 | 14.8±0.0 | 8.2±2.1 | 8.3±2.1 | 6.2±4.1 |

| | | | | | |
|---|---|---|---|---|---|
| *12-10 and 12-8 represent different parts of the scaffold_12:32514411-32671314 region; 54-8 represent scaffold_54:2219944-2303066; and 72-5 represent scaffold_72:1526932-1571665. | | | | | |

As evident from Table 3 and Figures 2, 3 and 9, mRNA levels in targeted integration clones were on average 1.7-, 1.3- and 1.4-fold higher for IgG1 LC, IgG1 HC and IgG4 HC, respectively, when compared to random integration clones. For IgG4, volumetric productivity in fed-batch process was significantly higher in targeted integration clones compared to random integration clones (Table 3 and Figure 10).

Since overall productivity of a cell line depends not only on mRNA expression levels of the transgene but - among other things - also on growth performance (incl. growth rate, maximum cell density, viability, etc), the productivity per cell per day (specific productivity; Qp) was assessed for every clone on day 4 of the process. Specific productivities of targeted int. clones were significantly higher compared to random integration clones. The increase in specific productivity was 1.5-fold on average for IgG1 and 1.6-fold on average for IgG4 targeted integration clones (Table 3; Figures 8 and 11).

Next, the transgene copy numbers per cell were also assessed for each clone. Targeted integration resulted in higher number of integrated transgene copies compared to random approach. Table 4 provides an analysis and comparison of the clones with single-copy integration. Still, specific productivity remained significantly higher in targeted integration clones versus random integration. Most measurements were 1.5-2 fold higher in targeted clones vs random clones.

Further assessed is the productivity and mRNA levels for each targeted region individually (see Table 5). Average specific productivity was increased in all target regions when compared to random integration, respectively.

## Claims

1. A method of producing an engineered Chinese Hamster Ovary (CHO) cell, the method comprising:
introducing into a CHO cell a construct for integration of an exogenous nucleic acid molecule into or within 500 kb upstream or downstream of an expression-enhancing sequence in the genome of the cell, the expression-enhancing sequence being at least 90% identical to a sequence selected from any one of SEQ ID NOs: 1-47.

2. The method according to claim 2, wherein the expression-enhancing sequence is selected from any one of SEQ ID NOs: 1-47.

3. The method according to any one of the preceding claims, wherein integration of the exogenous nucleic acid molecule is within 100 kb upstream or downstream of the expression-enhancing sequence.

4. The method according to any one of the preceding claims, wherein integration of the exogenous nucleic acid molecule is achieved by the CRISPR (Clustered Regulatory Interspaced Short Palindromic Repeats)/Cas9 method, TALEN (Transcription Activator-Like Effector Nuclease)-based method or ZFN (zinc-finger nuclease)-based method.

5. The method according to claim 4, wherein integration of the exogenous nucleic acid molecule is achieved by the CRISPR /Cas9 method.

6. The method according to any one of the preceding claims, wherein the exogenous nucleic acid molecule integrates at two or more integration sites in the genome of the cell.

7. The method according to any one of the preceding claims, wherein the exogenous nucleic acid encodes a polypeptide.

8. An engineered cell produced by the method according to any one of claims 1 to 7.

9. A method of producing a recombinant polypeptide, the method comprising:
(i) introducing into a CHO cell a construct for integration of an exogenous nucleic acid molecule into or within 500 kb upstream or downstream of an expression-enhancing sequence in the genome of the cell, the expression-enhancing sequence being at least 90% identical to a sequence selected from any one of SEQ ID NOs: 1-47, to produce an engineered cell,
(ii) culturing the engineered cell to recombinantly express the exogenous nucleic acid to produce a recombinant polypeptide encoded by the exogenous nucleic acid, and
(iii) isolating the recombinant polypeptide.

10. Use of a nucleic acid sequence being at least 90% identical to a sequence selected from any one of SEQ ID NOs: 1-47 for transgene expression.
